Europäisches Patentamt

European Patent Office

Office européen des brevets

(19)

(11) Publication number: **0 339 569**
**A2**

(12) # EUROPEAN PATENT APPLICATION

(21) Application number: **89107462.7**

(51) Int. Cl.⁴: **C12N 15/00 , C12P 21/02**

(22) Date of filing: **25.04.89**

The applicant has filed a statement in accordance with Rule 28 (4) EPC (issue of a sample only to an expert). Accession number(s) of the deposit(s): NRRL Y-15851, NRRL B-18114, NRRL B-15867, NRRL B-15868, NRRL B-15869, NRRL B-15890, NRRL B-18016.

(30) Priority: **25.04.88 US 186409**

(43) Date of publication of application:
**02.11.89 Bulletin 89/44**

(84) Designated Contracting States:
**AT BE CH DE ES FR GB GR IT LI LU NL SE**

(71) Applicant: **PHILLIPS PETROLEUM COMPANY**
**5th and Keeler**
**Bartlesville Oklahoma 74004(US)**

(72) Inventor: **Digan, Mary Ellen**
**178 Centre Street**
**Mountain View, CA 94041(US)**

(74) Representative: **Dost, Wolfgang,**
**Dr.rer.nat.,Dipl.-Chem. et al**
**Patent- & Rechtsanwälte Bardehle .**
**Pagenberg . Dost . Altenburg . Frohwitter .**
**Geissler & Partner Galileiplatz 1 Postfach 86**
**06 20**
**D-8000 München 86(DE)**

(54) **Expression of the HIV 24 KDA GAG protein in methylotrophic yeasts.**

(57) A process for the production of HIV 24 kDa gag protein. Also disclosed are novel DNA molecules and hosts transformed with these molecules.

PARTIAL RESTRICTION MAP OF EcoR I/Cla I
FRAGMENT OF HIV ENCODING p24

*FIG. 1*

EP 0 339 569 A2

Xerox Copy Centre

# EXPRESSION OF THE HIV 24 KDA GAG PROTEIN IN METHYLOTROPHIC YEASTS

## Field of Invention

This invention relates to the field of recombinant DNA biotechnology. In one aspect, this invention relates to a process for the expression of HIV 24 kDa gag protein in methylotrophic yeasts. In another aspect the present invention relates to novel DNA molecules and novel yeast strains transformed therewith.

## Background

The retrovirus HIV (formerly designated HTLV-III or LAV) has been demonstrated to be the caustive agent of acquired immune deficiency syndrome (AIDS). The viral genome of HIV contains three large open reading frames corresponding to the gag, pol and env genes. The gag gene codes for the core proteins of the virus, pol codes for the viral reverse transcriptase, protease and endonuclease and env codes for the viral glycoproteins.

The gag gene encodes a precursor which is processed into core proteins during virion maturation. The gag precursor is approximately 56 kDa and is proteolytically processed into three proteins of approximately 24, 16 and 14 kDa. The 24 kDa form of the gag core protein of the HIV retrovirus has been found to induce antibody formation in a large percentage of individuals who have previously been exposed to the virus.

Assays have been developed based on this observation which measure the presence or absence of antibodies to the HIV 24 kDa gag protein and constitute one method of assessing whether an individual has been exposed to HIV and may be a candidate for developing AIDS. To perform this assay, purified HIV 24 kDa gag protein is required as a diagnostic reagent. No readily available supply of HIV 24 kDa gag currently exists to meet the demand for protein reagent.

Similarly this protein potentially could also be utilized as a vaccine singly or in combination with other HIV viral proteins if the proteins could be produced in a safe economical manner.

Thus it would be a significant contribution to the art to develop an efficient process for the production of HIV 24 kDa gag protein.

Therefore, it is an object of this invention to provide a process for the production of HIV 24 kDa gag protein.

Yet another object of this invention is to provide novel vectors containing DNA sequences which code for the HIV 24 kDa gag protein.

A further object of this invention is to provide novel methylotrophic yeasts transformed with a vector or vectors capable of enhanced production of the HIV 24 kDa gag protein.

These and other objects of the invention will become apparent from the disclosure and claims herein provided.

## Summary of the Invention

In accordance with the present invention, I have discovered a process for the production of the HIV 24 kDa gag protein which comprises transforming a methylotrophic yeast with at least one vector compatible with the yeast expression cassette containing a structural gene for the HIV 24 kDa gag protein and culturing the resultant transformants under conditions suitable to obtain the production of HIV 24 kDa gag protein.

## Detailed Description of the Figures

Figure 1 provides a restriction map of the EcoRI/ClaI fragment of HIV which contains the 24 kDa gag gene of HIV.

Figure 2 provides a flow chart of the construction of plasmid pSL44A.

Figure 3 provides a representation of plasmid pSL44A. pSL44A is a derivative of pAO804 which has been modified by the insertion of an Fl-ori and the insertion of the HIV 24 kDa structural gene in the unique EcoRI site. P AO804 is available in NRRL B-18114 and may be recovered from the plasmid DNA as a 7.5 kb EcoRI fragment 7.

## Detailed Description

The HIV genome is well known in the art and has been sequenced by Ratner et al. Complete nucleotide sequence of the AIDS virus, HTLV-III Nature 313:277 (1985). The HIV 24 kDa gag protein nucleotide sequence begins at nucleotide 729 and ends at nucleotide site 1421. The nucleotide sequence utilized to code for the 24 kDa gag protein is provided in Table I (however, nucleotides between approximately 1010-1115 have not been sequenced to confirm their correspondence to the published sequence). The 24 kDa gag protein nucleotide sequence may therefore be obtained by reisolating the structural gene from HIV, by in vitro DNA synthesis or a combination of these techniques.

The structural gene for the HIV 24 kDa gag protein may be maintained in any suitable host vector system for propagation, maintenance or recombinant manipulation. Suitable host/vector systems for the practice of this invention include but are not limited to an E. coli host in combination with a pBR322 plasmid or a Pichia pastoris GS115 host in combination with pAO804 or pA0807.

Culturing the host strains listed above may be accomplished by any suitable means. General techniques for culturing hosts are already known in the art and any adaptation of these methods to the specific requirements of the strains used herein is well within the abilities of those skilled in the art.

Recovery of plasmid DNA from hosts can be accomplished by several techniques due to its compact size and closed spherical superhelical form. For example following the harvest, host cells may be pelleted by centrifugation and then resuspended and lysed. The lysate should be centrifuged to remove cell debris and the supernatant containing DNA retained. A PEG precipitation can then be performed to concentrate the DNA. The precipitated DNA may then be further treated using a density gradient centrifugation or a gel filtration technique to separate the plasmid DNA from the bacterial DNA. The techniques for achieving the separation alluded to above are well known in the art and numerous methods of performing these techniques are known.

## Table I

### Nucleotide Sequence of HIV 24 kDa gag Structural Gene

```
                               EcoRI
                               GAATTCATGCCTATAGTGCAGAACATCCAGGGGCAAATGGTAC
      481    _____|_____|_____|_____|_____|_____|  540

             ATCAGGCCATATCACCTAGAACTTTAAATGCATGGGTAAAAGTAGTAGAAGAGAAGGCTT
      541    _____|_____|_____|_____|_____|_____|  600

             TCAGCCCAGAAGTGATACCCATGTTTTCAGCATTATCAGAAGGAGCCACCCCACAAGATT
      601    _____|_____|_____|_____|_____|_____|  660

             TAAACACCATGCTAAACACAGTGGGGGGACATCAAGCAGCCATGCAAATGTTAAAAGAGA
      661    _____|_____|_____|_____|_____|_____|  720

                             P
                             s
                             t
                             I
             CCATCAATGAGGAAGCTGCAGAATGGGATAGAGTGCATCCAGTGCATGCAGGGCCTATCG
      721    _____|_____|_____|_____|_____|_____|  780

             CACCAGGCCAGATGAGAGAACCAAGGGGAAGTGACATAGCAGGAACTACTAGTACCCTTC
      781    _____|_____|_____|_____|_____|_____|  840

             AGGAACAAATAGGATGGATGACAAATAATCCACCTATCCCAGTAGGAGAAATTTATAAAA
      841    _____|_____|____·____|_____|_____|_____|  900

             GATGGATAATCCTGGGATTAAATAAAATAGTAAGGATGTATAGTCCTACCAGCATTCTGG
      901    _____|_____|_____|_____|_____|_____|  960

             ACATAAGACAAGGACCAAAGGAACCCTTTAGAGACTATGTAGACCGGTTCTATAAAACTC
      961    _____|_____|_____|_____|_____|_____|  1020

             TAAGAGCCGAGCAAGCTTCACAGGAAGTAAAAAAATTGGATGACAGAAACCTTGTTGGTCC
      1021   _____|_____|_____|_____|_____|_____|  1080

             AAAATGCGAACCCAGATTGTAAGACTATTTTAAAAGCATTGGGACCAGCAGCTACTCTAG
      1081   _____|_____|_____|_____|_____|_____|  1140

             AAGAAATGATGACAGCATGTCAGGGAGTGGGAGGACCCGGCCATAAAGCAAGAGTTTTGT
      1141   _____|_____|_____|_____|_____|_____|  1200

             EcoRI
             AAGAATTC
      1201   _____|
```

Nuclease digestion of the plasmid may be accomplished by choosing appropriate endonucleases which will cut the selected plasmid in such a way as to facilitate the recovery of the HIV gag structural gene. The endonucleases used will depend on the plasmid from which the HIV 24 kDa gag gene is to be excised.

Gel electrophoresis of DNA may be accomplished using numerous techniques. See P. G. Sealy and E. M. Southern, Gel Electrophoresis of Nucleic Acids - A Practical Approach (D. Rickwood and B. D. Hames, eds.) p. 39 (1982). Elution may also be accomplished using numerous techniques appropriate for the gel

4

involved, such as electroelution, diffusion, gel dissolution (agarose gels) or physical extrusion (agarose gels). It is additionally recognized that elution may not be necessary with some gels such as high-quality, low melting temperature agarose.

Once the fragment containing the HIV 24 kDa gag structural gene or fragments thereof is isolated, additional manipulations may be required before it is inserted in the vector. These manipulations may include, but are not limited to the addition of linkers or blunt-ending the fragment.

Following the isolation of the HIV gag structural gene, the gene is inserted into a suitable methylotrophic yeast vector such as a plasmid. Preferable vectors for the practice of this invention are those compatible with the Pichia genus and most preferably Pichia pastoris.

Plasmids have long been one of the basic elements employed in recombinant DNA technology. Plasmids are circular extrachromosomal double-stranded DNA found in microorganisms. Plasmids have been found to occur in single or multiple copies per cell. Included in plasmid DNA is the information required for plasmid reproduction, i.e. an origin of replication is included for bacterial replication. One or more means of phenotypically selecting the plasmid in transformed cells may also be included in the information encoded in the plasmid. Phenotypic or selective markers, such as antibiotic resistance genes or genes which complement defects in the host biochemical pathways, permit clones of the host cells which have been transformed to be recognized, selected; and maintained.

To express the HIV 24 kDa gag structural gene in methylotrophic yeast, the gene must be operably linked to a 5′ regulatory region and 3′ termination sequence, which forms the expression cassette which will be inserted into the host via a vector.

The following terms are defined herein for the purpose of clarification.

Operably linked--refers to a juxtaposition wherein the components are configured so as to perform their function.

Regulatory region--DNA sequences which respond to various stimuli and affect the rate of mRNA transcription.

3′ Termination sequence--sequences 3′ to the stop codon which function to stabilize the mRNA such as sequences which elicit polyadenylation.

"Pichia compatible" refers to DNA sequences which will perform their normal function in Pichia such as regulatory regions and 3′ termination sequences derived from Pichia.

Preferred for the practice of the present invention are integrative vectors, such as the linear site-specific integrative vector of Cregg, as described in European Application Serial Number 86114700.7. Such vectors comprise a serially arranged sequence of at least 1) a first insertable DNA fragment; 2) a selectable marker gene; and 3) a second insertable DNA fragment.

The first and second insertable DNA fragments are each at least about 200 nucleotides in length and have nucleotide sequences which are homologous to portions of the genomic DNA of species of the genus Pichia. The various components of the integrative vector are serially arranged forming a linear fragment of DNA such that the expression cassette and the selectable marker gene are positioned between the 3′ end of the first insertable DNA fragment and the 5′ end of the second insertable DNA fragment. The first and second insertable DNA fragments are oriented with respect to one another in the serially arranged linear fragment as they are so oriented in the parent genome.

Nucleotide sequences useful as the first and second insertable DNA fragments are nucleotide sequences which are homologous with separate portions of the native genomic site at which genomic modification is to occur. Thus, for example, if genomic modification is to occur at the locus of the alcohol oxidase gene, the first and second insertable DNA fragments employed will be sequences homologous with separate portions of the alcohol oxidase gene locus. For genomic modification in accordance with the present invention to occur, the two insertable DNA fragments must be oriented with respect to one another in the linear fragment in the same relative orientation as they exist in the parent genome. Examples of nucleotide sequences which could be used as first and second insertable DNA fragments are nucleotide sequences selected from the group consisting of the alcohol oxidase (AOX1) gene, dihydroxyacetone synthase (DHAS1) gene, p40 gene and HIS4 gene. The AOX1 gene, DHAS1; gene, p40 gene and HIS4 gene are contained in copending application serial number 780,102 incorporated herein by reference.

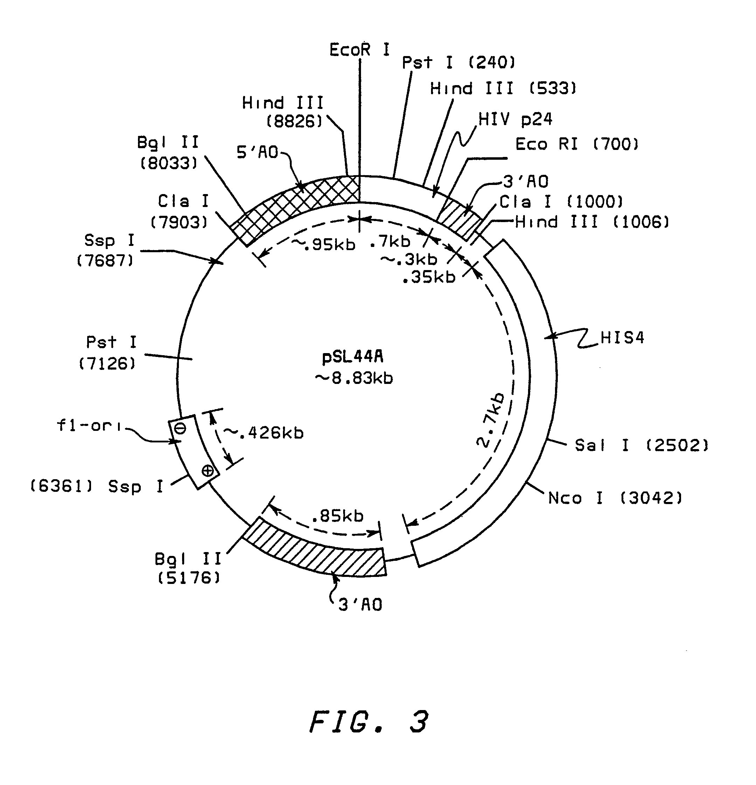

The first insertable DNA fragment may contain an operable regulatory region which may comprise the regulatory region utilized in the expression cassette. The use of the first insertable DNA fragment as the regulatory region for an expression cassette is a preferred embodiment of this invention. Figure 3 provides a diagram of a vector utilizing the first insertable DNA fragment as a regulatory region for a cassette.

Optionally as shown in Figure 3 an insertion site or sites and a 3′ termination sequence may be placed immediately 3′ to the first insertable DNA fragment. This conformation of the linear site-specific integrative vector has the additional advantage of providing a ready site for insertion of a structural gene without necessitating the addition of a compatible 3′ termination sequence.

It is also necessary to include at least one selectable marker gene in the DNA used to transform the host strain. This facilitates selection and isolation of those organisms which have incorporated the transforming DNA. The marker gene confers a phenotypic trait to the transformed organism which the host did not have, e.g., restoration of the ability to produce a specific amino acid where the untransformed host strain has a defect in the specific amino acid biosynthetic pathway or resistance to antibiotics and the like.

Exemplary selectable marker genes may be selected from the group consisting of the HIS4 gene and the ARG4 gene from Pichia pastoris and Saccharomyces cerevisiae, the invertase gene (SUC2) from Saccharomyces cerevisiae, or the G418 phosphotransferase gene from the E. coli transposable elements Tn601 or Tn903.

Those of skill in the art recognize that additional DNA sequences can also be incorporated into the vectors employed in the practice of the present invention, such as for example, bacterial plasmid DNA, bacteriophase DNA, and the like. Such sequences enable the amplification and maintenance of these vectors in bacterial hosts.

If the first insertable DNA fragment does not contain a regulatory region, a suitable regulatory region will need to be inserted operably linked to the structural gene, in order to provide an operable expression cassette. Similarly if no 3′ termination sequence is provided at the insertion site to complete the expression cassette, a 3′ termination sequence will have to be operably linked to the structural gene to be inserted.

Those skilled in the art are aware of numerous regulatory regions which have been characterized and could be employed in conjunction with methylotrophic yeasts. Exemplary regulatory regions include but are not limited to yeast regulatory regions selected from the group consisting of acid phosphatase, galactokinase, alcohol dehydrogenase, cytochrome c, alpha-mating factor and glyceraldehyde 3-phosphate dehydrogenase regulatory regions isolated from Saccharomyces cerevisiae; the primary alcohol oxidase (AOX1), dihydroxyacetone synthase (DAS1), the p40 regulatory regions, and the HIS4 regulatory region derived from Pichia pastoris and the like. Presently preferred regulatory regions employed in the practice of the present invention are those characterized by their ability to respond to methanol-containing media, such regulatory regions selected from the group consisting of AOX1, DHAS1, p40 which are disclosed in EP-A- 0183071.

The most preferred regulatory region for the practice of this invention is the AOX1 regulatory region.

3′ termination sequences may be utilized in the expression cassette or be part of the vector as discussed above. 3′ termination sequences may function to terminate, polyadenylate and/or stabilize the messenger RNA coded for by the structural gene when operably linked to a gene. A few examples of illustrative sources for 3′ termination sequences for the practice of this invention include but are not limited to the Saccharomyces cerevisiae, Hansenula polymorpha, and Pichia 3′ termination sequences. Preferred are those derived from Pichia pastoris such as those selected from the group consisting of the 3′ termination sequences of AOX1 gene, DHAS gene, p40 gene and HIS4 gene. And particularly preferred is the 3′ termination sequence of the AOX1 gene.

For the practice of the current invention it is currently preferred to use linear transformation vectors such as the clockwise Bgl II fragments of the constructs shown in Figure 3.

The insertion of the HIV 24 kDa gag structural gene into suitable vectors may be accomplished by any suitable technique which cleaves the chosen vector at an appropriate site or sites and results in at least one operable expression cassette containing the structural gene being present in the vector.

Ligation of HIV 24 kDa gag structural gene may be accomplished by any appropriate ligation technique such as utilizing T4 DNA ligase.

The initial selection, propagation, and optional amplification of the ligation mixture of the HIV 24 kDa gag structural gene and a vector is preferably performed by transforming the mixture into a bacterial host such as E. coli (although the ligation mixture could be transformed directly into a yeast host). Suitable transformation techniques for E. coli are well known in the art. Additionally, selection markers and bacterial origins of replication necessary for the maintenance of a vector in a bacterial host are also well known in the art.

The isolation and/or purification of the desired plasmid containing the HIV 24 kDa gag structural gene in an expression system may be accomplished by any suitable means for the separation of plasmid DNA from the host DNA.

Similarly the vectors formed by ligation may be tested preferably after propagation to verify the presence of the HIV 24 kDa gag gene and its operable linkage to a regulatory region and a 3′ termination sequence. This may be accomplished by a variety of techniques including but not limited to endonuclease digestion, gel electrophoresis, or endonuclease digestion-Southern hybridization.

Transformation of plasmids or linear vectors into yeast hosts may be accomplished by suitable transformation techniques including but not limited to those taught by Hinnen et al, Proc. Natl. Acad. Sci. 75, (1978) 1929; Ito et al., J. Bacteriol 153, (1983) 163; Cregg et al Mol. Cell Biol. 5 (1985) pg. 3376; or Sreekrishna et al, Gene, 59 (1987) pg. 115. Preferable for the practice of this invention is the transformation technique of Cregg.

The yeast host for transformation may be any suitable methylotrophic yeast. Methylotrophic yeast include but are not limited to yeast capable of growth on methanol selected from the genera consisting of Hansenula, Candida, Kloeckera, Pichia, Saccharomyces, Torulopsis and Rhodotorula. A list of specific species which are exemplary of this class of yeasts may be found in C. Anthony, The Biochemistry of Methylotrophs, 269, (1982). Presently preferred are methylotrophic yeasts of the genus Pichia such as the auxotrophic Pichia pastoris GS115 (NRRL Y-15851). Auxotrophic methylotrophic yeasts are also advantageous to the practice of this invention for their ease of selection. It is recognized that wild type methylotrophic yeast strains may be employed with equal success if a suitable transforming marker gene is selected, such as the use of SUC2 to transform Pichia pastoris to a strain capable of growth on sucrose or an antibiotic resistance marker is employed, such as G418.

Transformed methylotrophic yeast cells can be selected for using appropriate techniques including but not limited to culturing previously auxotrophic cells after transformation in the absence of a biochemical product required (due to the cell's auxotrophy), screening by the detection of a new phenotype ("methanol utilization +/-, Mut +/-"), or culturing in the presence of an antibiotic which is toxic to the yeast in the absence of a resistance gene contained in the transformant.

Isolated transformed methylotrophic yeast cells are cultured by appropriate fermentation techniques such as shake flask fermentation, high density fermentation or the techniques as disclosed by Cregg, et al. High-Level Expression and Efficient Assembly of Hepatitis B Surface Antigen in the Methylotrophic Yeast, Pichia Pastoris, 5 Bio/Technology 479 (1987).

Expression may be accomplished by methods appropriate to the regulatory region employed. Preferably if methanol responsive regulatory regions are utilized, the induction of expression may be accomplished by exposing the transformed cells in a nutrient media to an appropriate alcohol for the regulatory region employed.

The HIV 24 kDa gag protein may be recovered in a crude form by lysing transformed cells which have been induced for a sufficient period, using standard techniques such as bead milling, followed by centrifugation sufficient to remove cellular debris. A preferred method of recovery is included in Example VI.

The following non-limiting examples are provided to further illustrate the practice of this invention.


## Examples


General information pertinent to the Examples:


## Strains

Pichia pastoris GS115 (HIS4) [NRRL Y-15851] was the host yeast strain used in these examples.


## Buffers, and Solutions, and Media

The buffers and solutions employed in the following examples have the compositions given below:

| | |
|---|---|
| dH$_2$O | deionized H$_2$O that has been treated with a milli-Q (Millipore) reagent water system. |
| 1M Tris buffer | 121.1 g Tris base in 800 mL of H$_2$O; adjust pH to the desired value by adding concentrated (35%) aqueous HCl; allow solution to cool to room temperature before final pH adjustment, dilute to a final volume of 1L. |
| TE buffer | 1.0 mM EDTA<br>in 0.01 M (pH 8.0) Tris buffer |
| SED | 1 M sorbitol<br>25 mM EDTA<br>50 mM DTT<br>--adjust to pH 8 |
| SCE | 9.1g sorbitol<br>1.47g Sodium citrate<br>0.168g EDTA<br>--pH to 5.8 with HCl in 50 ml dH$_2$O and autoclave |
| CaS | 1 M sorbitol<br>10 mM CaCl$_2$<br>--filter sterilize |
| PEG Solution | 20% polyethylene glycol-3350<br>10 mM CaCl$_2$<br>10 mM Tris-HCl (pH 7.4)<br>--filter sterilize |

| | |
|---|---|
| Solution A | 0.2M Tris-HCl (pH 7.5)<br>0.1M $MgCl_2$<br>0.5M NaCl<br>0.01M dithiothreitol (DTT) |
| Solution B | 0.2M Tris-HCl (pH 7.5)<br>0.1M $MgCl_2$<br>0.1M DTT |
| Solution C (keep on ice) | 4µl solution B<br>4µl 10mM dATP<br>4µl 10mM dTTP<br>4µl 10mM dGTP<br>4µl 10mM dCTP<br>4µl 10mM ATP<br>6µl $T_4$ ligase (2U µl)<br>12µl $H_2O$<br>Recipe for Solution C was modified from Zoller & Smith |
| 20X SSPE | 4.4g NaOH<br>7.4g $Na_2EDTA$<br>27.6g $NaH_2PO_4 \cdot H_2O$<br>210g NaCl<br>--pH adjusted to 7.5-8.0 with NaOH<br>--$H_2O$ to 1 liter |
| 50X Denhardt's | 5g Ficoll 400<br>5g Polyvinylpyrolidine<br>5g BSA Fraction V<br>$H_2O$ to 500 ml |
| 20X SSC | 175.3g NaCl<br>88.2g sodium citrate<br>--pH to 7.0 with NaOH<br>--$H_2O$ to 1 liter |
| medium composition (7.0-liter) | 480g glycerol<br>40mg biotin<br>134ml $H_3PO_4$ (85%)<br>5.8g $CaSO_4 \cdot 2H_2O$<br>92g $K_2SO_4$<br>75g $MgSO_4 \cdot 7H_2O$<br>21g KOH |
| IM1 Trace Salts Solution, 1 liter | 0.06g Cupric Sulfate·$5H_2O$<br>0.08g Potassium Iodide<br>0.30g Manganese Sulfate·$H_2O$<br>0.20g Sodium Molybdate<br>0.02g Boric Acid<br>2.00g Zinc Sulfate·$H_2O$<br>4.8g Ferric Chloride·$H_2O$<br>5.00ml/liter Sulfuric Acid |

```
10X Transfer Buffer          96.8g Trizma Base
                             9.74g glycine
                             water to 1 liter

Transfer Buffer for          500mls 10X Transfer Buffer
Tank                         1000 mls methanol
                             3500 mls water

Western Buffer -             2.5g gelatin put in solution by microwaving
for 1 liter                  first in 100 mls water
                             100mls 10X PBS
Regeneration Agar            1ml 50% Tween-20
                             4mls 5% sodium azide
c 6 M KCl                    dH₂O to 1 liter
2% Agar
```

0.6 M KCl
2% Agar

Example I

Creation of pAO807

The pAO804 plasmid is available in an E. coli host from the Northern Regional Research Center of the United States Department of Agriculture, Peoria, Illinois accession number B-18114). pAO804 is recovered by isolating the plasmid DNA, digesting with EcoRI, gel electrophoresing to recover the ~7.5 kb fragment which is linear pAO804 cut at its unique EcoRI site.

I. Preparation of fl-ori DNA

fl bacteriophage DNA (50 ug) was digested with 50 units of Rsa I and Dra I (according to manufacturer's directions) to release the ~458 bp DNA fragment containing the fl origin of replication (ori). The digestion mixture was extracted with an equal volume of phenol:chloroform (V/V) followed by extracting the aqueous layer with an equal volume of chloroform and finally the DNA in the aqueous phase was precipitated by adjusting the NaCl concentration to 0.2M and adding 2.5 volumes of absolute ethanol. The mixture was allowed to stand on ice (4°C) for 10 minutes and the DNA precipitate was collected by centrifugation for 30 minutes at 10,000 xg in a microfuge at 4°C.

The DNA pellet was washed 2 times with 70% aqueous ethanol. The washed pellet was vacuum dried and dissolved in 25 ul of TE buffer. This DNA was electrophoresed on 1.5% agarose gel and the gel portion containing the ~458 bp fl-ori fragment was excised out and the DNA in the gel was electroeluted onto DE81 (Watman) paper and eluted from the paper in 1M NaCl. The DNA solution was precipitated as detailed above and the DNA precipitate was dissolved in 25 ul of TE buffer (fl-ori fragment).

2. Cloning of fl-ori into Dra I sites of pBR322:

pBR322 (2 ug) was partially digested with 2 units Dra I (according to manufacturer instructions). The reaction was terminated by phenol:chloroform extraction followed by precipitation of DNA as detailed in step 1 above. The DNA pellet was dissolved in 20 ul of TE buffer. About 100 ng of this DNA was ligated with 100 ng of fl-ori fragment (step 1) in 20 ul of ligation buffer by incubating at 14°C for overnight with 1 unit of T4 DNA ligase. The ligation was terminated by heating to 70°C for 10 minutes and then used to transform E. coli strain JM103 to obtain pBRfl-ori which contains fl-ori cloned into the Dra I sites (nucleotide positions 3232 and 3251) of pBR322.

3. Creation of pAO807:

pBRfl-ori (10 ug) was digested for 4 hours at 37°C with 10 units each of Pst I and Nde I. The digested DNA was phenol:chloroform extracted, precipitated and dissolved in 25 ul of TE buffer as detailed in step 1 above. This material was electrophoresed on a 1.2% agarose gel and the Nde I - Pst I fragment (approximately 0.8 kb) containing the fl-ori was isolated and dissolved in 20 ul of TE buffer as detailed in step 1 above. About 100 ng of this DNA was mixed with 100 ng of pAO804 that had been digested with Pst I and Nde I and phosphatase treated. This mixture was ligated in 20 ul of ligation buffer by incubating for overnight at 14°C with 1 unit of T4 DNA ligase. The ligation reaction was terminated by heating at 70°C for 10 minutes. This DNA was used to transform E. coli strain JM103 to obtain pAO807.

Example II

Construction of Vector pSL34A

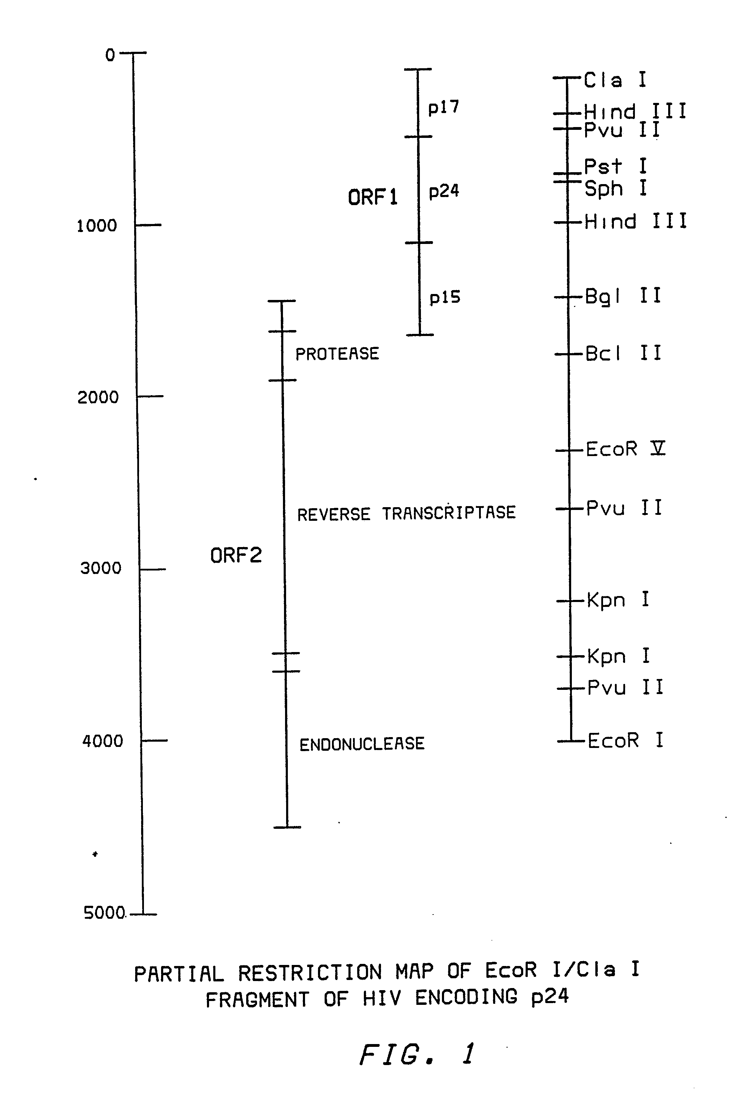

An EcoR I/Cla I fragment from the HIV virus containing the 24 kDa gag sequence, and other HIV sequence comprising 3.85 kb was isolated (see Figure 1). 25 ng of this fragment was ligated overnight at 14°C utilizing 1 unit os T4 ligase (Boehringer Mannheim) with 10 ng of pBR322 DNA in 20 μl of ligase buffer (Maniatis et al. 1982) which had been digested with a 4:1 excess of EcoRI and ClaI endonuclease according to the manufacturers directions (all endonucleases acquired from New England Biolabs or Boehringer Mannheim), and treated with calf intestinal alkaline phosphatase.

Competent cells were prepared by growing 100 ml culture of bacteria MC1061 on LB media to early log phase. The cells were then pelleted by centrifugation at 3000 rpm for 5 minutes at 4°C in a IEC DPR-6000 centrifuge. The cells were then resuspended in 40 ml of CG solution (50mM $CaCl_2$ and 10% glycerol) and put on ice for 15-20 minutes. The cells were pelleted by centrifugation as before and resuspended in 5 ml of CG. Aliquots of .45 ml/tube were placed in 1.5 ml Eppendorf tubes, quick frozen in liquid nitrogen and stored at -70°C. 200 μl of cells were used in each transformation with a final concentration equivalent to 6-8 $A_{600}$ units.

The ligation mixture of pBR322 and the HIV EcoRI/ClaI was used to transform 200 μl of competent MC1061 cells. The cells and ligation mixture were mixed and placed on ice for 20 minutes then incubated at 37°C for 2 minutes. The mixture was then left at room temperature for 10 minutes. The transformation mixture was then plated on selection media (LB media plus 50 μg/ml of ampicillin). The plates were incubated overnight at 37°C.

Ampicillin resistant colonies were identified and minipreps were performed on each resistant colony following the technique of Maniatis utilizing alkaline lysis. The plasmid DNA recovered was digested with a 4:1 excess of EcoRI and ClaI. The fragments were then run on a 0.8% agrose gel in TBE buffer. Colonies which had a 3.85 kb ClaI/EcoRI fragment appearing on the gel were designated p SL32 and pooled.

An oligonucleotide adapter was synthesized by an Applied Biosystems Model 380 gene machine which utilizes cyanoethylphosphoramidite chemistry. The adapter was designed to provide an EcoRI site to facilitate insertion of the 3.85 kb ClaI/EcoRI fragment into the unique EcoRI site of expression vector pAO807. The oligonucleotide adapter synthesized had the following nucleotide sequence:

```
5' CGATGAATTCATGGGTGCGAGAGCGTCAGTATTAAGCGGGGGAGAATTAGA 3'
3'    TACTTAAGTACCCACGCTCTCGCAGTCATAATTCGCCCCCTCTTAATCTAGC 5'
```

Each strand was synthesized individually and 5μg of both oligonucleotides were combined in a kinase reaction performed using the method of Maniatis and incubated at 37° for 1 hour. After the incubation, the mix was heated at 65°C for five minutes in a water bath which was then turned off and allowed to slow-cool to room temperature. 10 μg of plasmid pSL32 was digested with ClaI, and the ends were phosphatase-treated by the method of Maniatis. 10-25 ng of the phosphatased vector was ligated utilizing approximately 1 unit of T4 ligase with 15-150 ng of kinased, annealed oligonucleotide adapters which were present in a 1:4 to 1:10 molar excess. This reaction mixture was then used to transform competent MC1061 cells which were plated on selective media (these techniques were performed as previously described above). Minipreps were performed on the ampicillin resistant colonies and each colony was digested with EcoRI; those having the adapters would release a ~3.85 kb fragment. One colony having the insert was designated pSL33.

A large scale prep of a plasmid pSL33 was made by growing the MC1061 cell transformed with pSL33 to stationary phase in 100 ml of selective media (LB + 50 μg/ml ampicillin). The cells were then centrifuged at 10,000 rpms using a GSA rotor in a Sorvall RC-5B centrifuge for 10 minutes. The pellet was resuspended in 25 ml of a GET solution (comprising 50mM glucose, 10mM EDTA, 25 mM TRIS•Cl, pH 8) plus .5 ml of 10% lysozyme) and incubated at room temperature for 1-5 minutes. 50 ml of 1% SDS solution and 0.2N NaOH were added and were mixed by inversion to resuspend the pellet. This was followed by the addition of 38 ml of 3M potassium acetate (pH 4.5) which was mixed therein by inversion. This mixture was chilled 15 minutes in ice water. The chilled mixture was then centrifuged again for 10 minutes at 10,000 rpms using a GSA rotor in a Sorvall RC-58 centrifuge. The supernatant was then poured through a gauze pad into a new centrifuge bottle. 12 grams of PEG 8000 (polyethylene glycol) was added to the supernatant, and the resultant mixture chilled for 30 minutes in ice water. The chilled mixture was then centrifuged as previously described (10,000 rpms, 10 minutes, GSA rotor, Sorvall RC-58). The centrifuge tube was then decanted thoroughly and the pellet dissolved in 9 mls TE buffer (10mM TRIS (pH 8.0) and 1mM EDTA). 9 grams of CsCl and 0.45 mls of 1% ethidium bromide were added to the dissolved pellet. This mixture was then sealed in a tube and inserted in a T175 rotor and centrifuged at 55,000 rpms for 16 hours at 18°C in a Beckman L8-70 ultracentrifuge. The DNA bands were removed from the CsCl and extracted 3 or 4 times with sec-butanol saturated with NaCl. Two times the original volumes of water was added. Three times the original volume of isopropanol was added to this mixture. The mixture is chilled at -20°C for several hours or overnight, or chilled on dry ice until frozen. The DNA was then pelleted by centrifugation, for 20 minutes at 11,000 rpms, in a HB-4 rotor using a Sorvall RC-58 centrifuge. The supernatant was poured off and the pellet resuspended in 0.45 ml TE buffer then transferred to an Eppendorf tube. .05 ml of 3M sodium acetate and 1 ml of ethanol were added to the resuspended pellet, and it was chilled on dry ice until frozen. The pellet was then thawed and centrifuged in a microcentrifuge in a cold room (4°C). The thawed pellet was then dissolved in 200 μl of TE buffer. The concentration of the resultant DNA was determined by taking absorbance readings at 260 nm and estimating the concentration (1 equaled 50 μg/ml).

25 μg of pSL33 was digested with a 4:1 excess of EcoRI as described above. The digestion mixture was electrophoresed on .7% agarose gel in TBE buffer (10X TBE consists of 108 gr TRIS base, 55 gr boric acid 6.72 gr disodium EDTA (resulting pH 8.1-8.2)). The 3.85 kb EcoRI fragment generated by this digestion was cut out of the gel, placed in a dialysis bag and electroeluted in TBE buffer. The electroeluted fragment was then precipitated with 2 volumes of ethanol and phenol chloroform extracted. 12.5-125ng of this 3.85 kb EcoRI fragment were then ligated into 10 ng of EcoRI cut and phosphatased treated plasmid pAO807 The ligation was performed using the ligase buffer of Maniatis.

The ligation mixture was then transformed into 200 ul of competent JM103 cells.

Competent JM103 cells were prepared by seeding 5 ml of supplemented M9 media, Zollar and Smith, Methods in Enzymology 100:468, 490 (1983) with JM103 and culturing at 37°C with shaking until cells reach stationary phase. 50 μl of the stationary phase JM103 was added to 50 ml of LB media and cultured at 37°C for 2 hours or until OD600 was .3. The cells were harvested and centrifuged in an IEC DPR6000 at 3000 rpms for 5 minutes at 4°C. The cells were then resuspended in 25ml of 50mM CaCl2 and placed on ice for 15 minutes. At the end of 15 minutes the cells were again centrifuged in an IEC DPR6000 at 3000 rpms for 5 minutes at 4°C. The cells were then resuspended in 2.5 mls of 50mM CaCl2 and kept on ice until use (cells may only be retained 2-3 hours before use).

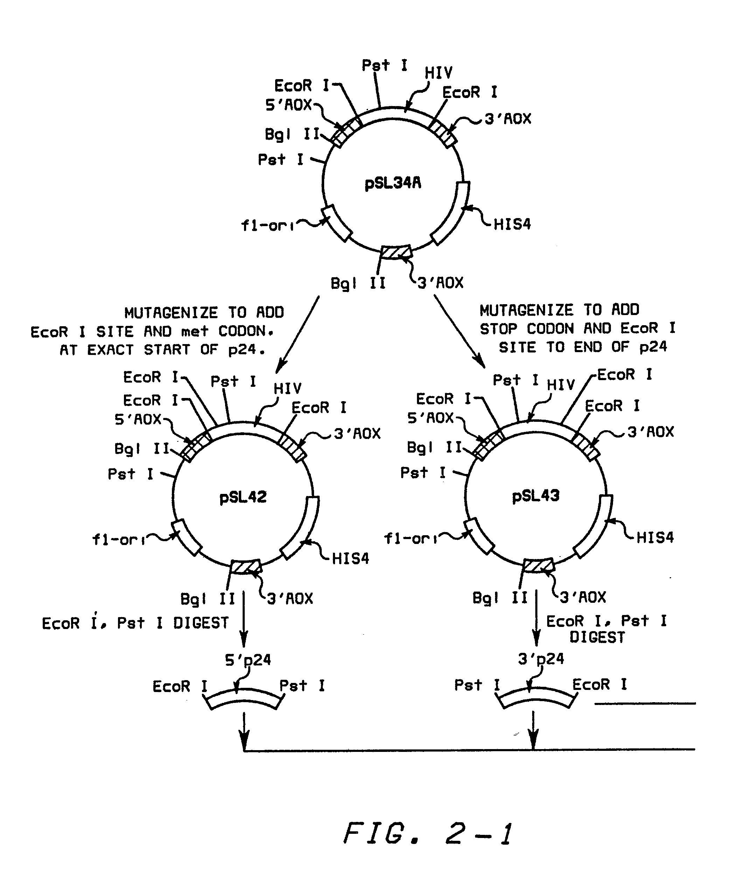

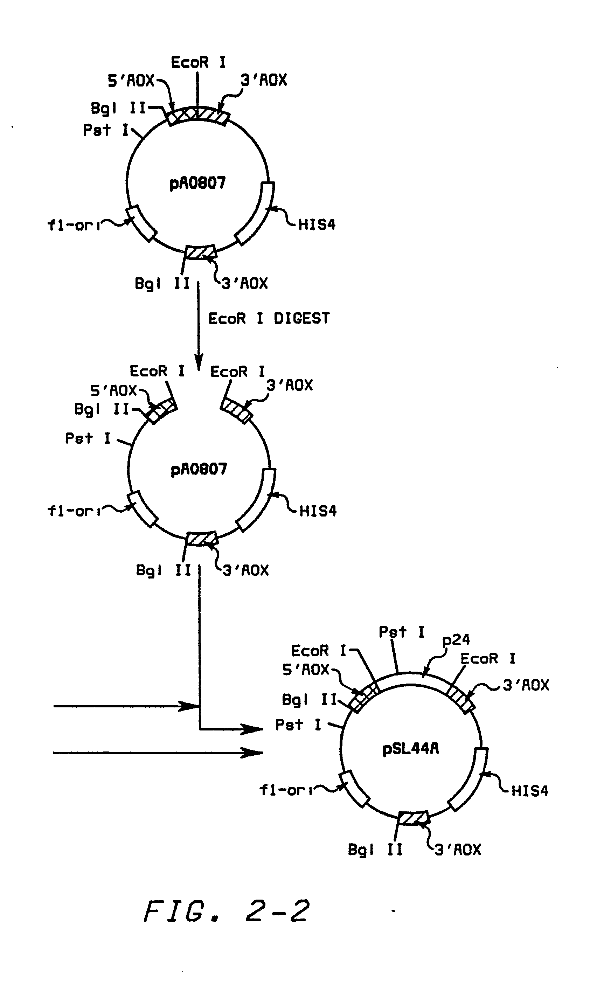

The transformation protocol for JM103 is the same as that previously used for MC1061. The cells were plated on LB media containing ampicillin (as previously described for MC1061) and colonies resistant to ampicillin selected. Minipreps were performed using the protocol of Maniatis for alkaline lysis. The DNA obtained from the minipreps was digested with EcoRI and run on a .7% agarose gel. A colony which generated a ~3.85 kb fragment was selected and designated pSL34A, which is shown in Figure 2. The orientation of this plasmid was also determined by Cla1 digest.

## Example III

## Construction of pSL44A

I. Oligonucleotide Directed Mutagenses

Plasmid pSL34A was subjected to independent mutagenesis to 1) add an EcoRI site and initial methionine at the 5' end of the gag gene and 2) to add a stop codon and an EcoRI site at the 3'-end of the gag gene yielding two derivative pSL34A plasmids (pSL42 and pSL43, see Figure 2). Template DNA was prepared from the pSL34A transformants by resuspending a colony of JM103 cells transformed to ampicillin resistance (by the pSL34A plasmid) in 2 ml of LB plus 100 μg/ml ampicillin and growing at 37°C for 4-5 hours. 0.5 ml of this saturated culture was inoculated into 50 ml of LB + amp and grown to an $OD_{600}$ = 0.3. It was then ready for superinfection with helper phage, the preparation of which is described below.

Phage stock from the helper phase R408 is prepared by placing 0.2 ml of an overnight culture of JM103 in supplemented M9 media plus $7 \times 10^8$ R408 plus 100 μl of LB broth in a small sterile tube. The tube is incubated at 37°C for 15 minutes without shaking and then 2.5 ml of 0.7% agar in LB is added and the mixture is poured onto the surface of an LB plate which was made the previous day. The plates are grown for six hours at 37°C and then overlayered with 5 ml of LB and allowed to soak in the coldroom overnight or at room temperature with slow shaking on a rotary shaker for 45 minutes. The supernatant is withdrawn, centrifuged using a Sorvall centrifuge and HB4 or SS34 head at 8,000 rpm for 20 minutes, and the supernatant from this spin is withdrawn to a new sterile 50 ml conical tube. The tube is heated for 30 minutes at 65°C. The stock should be stored at 4°C.

Titering of the phase is performed by making serial dilution of the phage stock into 1 ml of LB at concentrations of $10^{-2}$, $10^{-4}$, $10^{-6}$, $10^{-8}$ and $10^{-9}$. Lawns of bacteria for titering are prepared by plating on an LB plate 0.2 ml of an overnight culture of JM103 plus 2.5 ml of 0.7% agar, and letting the plate sit on the bench with the lid cracked for five minutes. 20 μl of each dilution are spotted onto the plate with glass micropipettes, and the plates are incubated at 37°C overnight. After overnight growth, the presence of 10 plaques in 20 μl of a 10-9 dilution would indicate a titer of $5 \times 10^{11}$. This is the expected value for the number of phase used to make the stock and is a useful titer for superinfection of plasmid cultures for single-stand production.

The $OD_{600}$ = 0.3 culture was superinfected with helper phage R408 [Russel et al (1986) Gene 45:333-338] at a multiplicity of infection of 10-20. The superinfected culture was then centrifuged twice at 10,000 rpms for 10 minutes in 30 ml corex tubes using a Sorval RC-5B centrifuge. The supernatant was transferred to fresh corex tubes and 7 ml of 20% PEG 6000 with 2.5M NaCl was added thereto. The mixture was incubated overnight at 4°C. The mixture was next centrifuged for 10 minutes at 10,000 rpms using a Sorvall RC-5B centrifuge and the supernatant was discarded. The pellet was resuspended in 2 ml of TE and extracted with phenol; phenol:chloroform and then ether. 1/10 the volume of 8M lithium chloride and 2 volumes ethanol were added to the mixture. The mixture was incubated at -20°C overnight. After overnight incubation the mixture was centrifuged for 10 minutes at 10,000 rpms in a Sorvall RC-5B centrifuge. The supernatant was discarded and the pellet rinsed with ethanol. The pellet was resuspended in 0.5 ml 10mM Tris•HCl.

The 5' oligonucleotide used for priming was of sequence:
5'-AGC AGT CAG GTC AGC GAA TTC ATG CCT ATA GTG CAG AAC- 3'
The 3' oligonucleotide used for priming was of sequence:
5'- AAG GCA AGA GTT TTG TAA GAA TTC ATG AGC CAA GTA ACA- 3'
Synthetic oligonucleotides were again made using an Applied Biosystems DNA synthesizer, model 380A, and cyanoethylphosphoramidite chemistry as previously described. The 5' mutagenesis was performed using template DNA from plasmid pSL34A according to the following protocol, yielding plasmid pSL42. The 3' mutagenesis was performed using template DNA from pSL34A according to the following portocol, yielding plasmid pSL43.

One nmole of the mutagenic oligonucleotide was kinased with non-radioactive ATP by conventional protocols (Maniatis, et al, 1982). The sequence of these oligonucleotides is given above. Forty pmoles of each kinased oligonucleotide was separately annealed to two pmoles of template DNA from pSL34A in Solution A (0.2M Tris-HCl (pH7.5), 0.5M NaCl, 0.1M MgCl2 and 0.01M dithiothreitol (DTT)) by boiling the

13

reaction mix for two minutes, incubating the mix for 5 minutes at 65°C, and slow cooling the reaction to room temperature by turning off the water bath. 2.5 μl of the Klenow fragment of E. coli DNA polymerase I (New England Biolabs) and 25 μl of Solution C were added to each tube.

The tubes were then incubated on the bench for 5 minutes and transferred to 16°C overnight.

Mutagenesis of the 3′ sequence was performed independently using the same protocol on separate DNA tempate from pSL34A.

II. Transformation into Bacteria and Colony Screening

The next morning, competent cells of the female bacterial strain, MC1061, were transformed with 0.5, 2 and 5 μl of a 1:40 dilution of the extension reaction, using the methods for transformation and for preparing competent cells given above. Five plates of each dilution were plated. After overnight incubation at 37°C, DNA from bacterial colonies was transferred to nitrocellulose filters using the methods on Grunstein and Hogness; and the colonies were hybridized with a screening oligonucleotide, which had been rendered radioactive by kinase-labelling in the presence of radioactive gamma-ATP by the method of (Zollar and Smith supra et 492). The sequence of the oligonucleotide used for screening the N-terminal mutagenesis was:

5′ -GTC AGC GAA TTC ATG CCT ATA- 3′

and the sequence of the oligonucleotide used for screening the C-terminal mutagenesis was:

5′ -GTT TTG TAA GAA TTC ATG AGC- 3′

Both oligonucleotides were synthesized as previously described except the trityl group was removed from the N-terminal oligo by the machine and the sample was desalted using a Sephadex G-40 medium column (rather than HPLC).

Hybridization of the radioactive oligonucleotides was conducted at 42°C in 5 x SSPE, 10 x Denhardt's and 0.5% SDS. Filters were prehybridized prior to hybridization in the same solution at 65°C for 30 minutes, and the solution was changed for hybridization. Labelled oligonucleotides were used in the hybridization at 10⁶ cpm/ml. The filter was washed at room temperature in three 15 minute washes in 6 x SSC, and one 15 minute wash at 42°C in 6 x SSC. After exposure to X-ray film (Kodak, X-Omat AR), eight putative single positive colonies were picked for each mutagenesis; DNA from the colonies was prepared using a standard bacterial miniprep protocol (Maniatis) and the DNA was checked for the presence of a new EcoRI site by digesting with this EcoRI and running the digests on polyacrylamide gels. Four and two, respectively, for the N- and C-terminal ends, of the bacterial minipreps confirmed the presence of the new EcoRI site. One of each of these positives was grown up for a large preparation of plasmid DNA, using the method of Maniatis and 1, 5, 10 and 20 μl of a 1:40 dilution of the miniprep DNA from each of these same positives was used to transform the bacterial strain, JM103 to ampicillin-resistance.

III. Preparation of Template DNA and Confirmation by Sequencing

Three colonies from each of the 20 μl dilution plates were used to prepare single-stranded template DNA by the methods above (involving polyethylene glycol precipitation of the supernatant of superinfected JM103 cells, subsequent deproteinization with phenol and chloroform and ethanol precipitation of the DNA). The sequence of the N-terminal mutagenesis was confirmed using oligonucleotides of sequence:

5′ -CAC CAA GGA AGC TTT AGA C- 3′

and of C-terminal mutagenesis was confirmed using oligonucleotide of sequence:

5′ -CGA ACC CAG ATT GTA AGA CTA- 3′

using the chain termination method of Sanger (1977). After pSL44 was made by ligation of the EcoRI-PstI HIV fragments from pSL42 and pSL43 into the EcoRI site of pAO807, template was prepared from pSL44. The sequence through the PstI site of this template was confirmed using oligonucleotide:

5′ -CAA GCA GCC ATG CAA ATG TT- 3′

To construct pSL44A, 10μg plasmid DNA from pSL42 and pSL43 were digested with EcoRI and PstI in 4:1 excess. The 5′ p24 fragment was an ~240bp EcoRI-PstI fragment; the 3′ p24 fragment was an ~460bp Pst-EcoRI fragment. 10μg of plasmid pAO807 was digested with EcoRI, the ends were phosphatase treated, and the vector plus the two p24-encoding fragments were joined in a three-way ligation. The ligation mix was transformed into MC1061 cells and ampr cells were identified. Plasmid minipreps were performed on the transformants and the correct plasmid was identified by digestion and electrophoresis on .8% agarose; it was called pSL44A (see Figure 3). Fragments were recovered from 5% polyacrylamide gels by diffusion from the DNA-containing gel pieces.

Example IV

Transformation of Pichia pastoris

I. Vector Preparation

20 μg of plasmid pSL44 was digested to completion with BglII.

The complete digestion of the plasmid was determined by .8% agarose gel electrophoresis. The digestion mixture was used to transform Pichia pastoris strain GS115 (his4-) deposited with the Northern Regional Research Center of the U.S. Department of Agriculture, accession number NRRL Y-15851. Transformation with vectors containing the histidinol dehydrogenase gene will complement this defect in the histidine pathway, changing the GS115 transformed cells to His[+]. Screening for transformants may therefore be easily accomplished by culturing the cells after transformation in a growth environment lacking histidine and recovering the cells capable of growing under this condition.

II. Cell Growth

Pichia pastoris GS115 (NRRL Y-15851) was inoculated into about 10 ml of YPD medium and shake cultured at 30° C for 12-20 hours. 100 ml of YPD medium was inoculated with seed culture to give an $OD_{600}$ of about 0.001. The medium was cultured in a shake flask at 30° C for about 12-20 hours. The culture was harvested when the $OD_{600}$ was about 0.2-0.3 (after approximately 16-20 hours) by centrifugation at 3000 rpms for 5 minutes using IEC DPR 6000 centrifuge.

III. Preparation of Spheroplasts

The cells were washed once in 10 ml of sterile water, and then centrifuged at 30000 rpm for 5 minutes. (Centrifugation is performed afte each cell wash at 3000 rpms for 5 minutes using a IEC DPR 6000 unless otherwise indicated.) The cells were then washed once in 10 ml of freshly prepared SED, once in 10 ml of sterile 1M sorbitol, and finally resuspended in 10 ml of SCE buffer. 10 μl of 4 mg/ml solution of Zymolyase (100,000 units/g obtained from Miles Laboratories) was added. The cells were then incubated at 30° C for about 60 minutes. The spheroplasts were washed twice in 10 ml of sterile 1M sorbitol by centrifugation at 1500 rpms for 10 minutes. 10 ml of sterile CaS was used as a final cell wash, and the cells were centrifuged again at 1500 rpms for 10 minutes and resuspended in 0.6 ml of CaS.

IV. Transformation

GS115 cells were transformed with .0.5, 1, 5 and 10 μg of the plasmid DNA digest. DNA samples were added to 100 μl of spheroplasts and incubated at room temperature for about 20 minutes. 1 ml of PEG solution was added to each sample and incubated at room temperature for about 15 minutes.

V. Regeneration of Spheroplasts

A bottom agar layer of 10 ml of regeneration agar was poured per plate at least 30 minutes before transformation samples were ready. In addition, 10 ml aliquots of regeneration agar were distributed to 50 ml conical bottom corning tubes in a 55° C bath. Aliquots of 10 or 990 μl of the transformed sample was added to the 10 ml aliquots of melted regeneration agar held at 55° C and poured onto plates containing the solid 10 ml bottom agar layer. The plates were incubated at 30° C for 3-5 days.

VI. Selection of Transformants

Transformants were selected for by culturing on regenerative agar, a media lacking histidine. His + colonies were identifed and subsequently screened for the appropriate Mut⁻ phenotype in the following manner. Transformants were pooled by scraping the surface of the plate in the presence of sterile distilled water, and sonicated at low output for 15 seconds. They were subsequently diluted to an $A_{600}$ = 0.1 and plated at dilutions of $10^{-3}$ and $10^{-4}$ in duplicate onto minimal plates containing 0.2% glucose as carbon source and incubated at 30°C for 1.5-2 days. They were then replica plated onto minimal plates with 0.5% methanol as a carbon source. After overnight incubation at 30°C, it was apparent that 18/75 (24%) of the transformants were growing more slowly on methanol than the rest of the transformants. The pattern of fragments on a genomic Southern blot predicted by replacement of the AOX1 gene by the BglII fragment from pSL44 was demonstrated in several of these strains. One of these strains was designated G-GAG44.

Example V

Fermentation of G-GAG44

A 10L fermentation Run #358 was performed as follows. Five hundred ml of buffered Nitrogen Base (YNB) -2% glycerol in a Fernback Flask were inoculated from a seed culture or a minimal glucose plate of the culture. After one day of shaking at 200 rpm and 30°C, the inoculum was seeded into 7 liters of minimal medium containing 480g glycerol, 40 mg biotin, and 40 ml trace salts solution. The fermentor was maintained at 30°C and pH 5.5 while the culture grew in batch mode until the glycerol was exhausted (about 24 hours). The pH was controlled by the addition of $NH_3$ gas. Glycerol exhaustion was noted by a sharp decline in the $CO_2$ evolution and sharp rise in the dissolved oxygen (or decrease in oxygen uptake rate). A methanol feed was initiated at 9 ml/h to bring the fermentor level up to ~0.5% MeOH, and adjusted, based on the actual MeOH consumption rate, to maintain this level. Forty ml aliquots of trace salts were added to approximately two day intervals to maintain the methanol consumption rate.

Example VI

Cell Breakage and Extraction

Cells were removed from the fermentation broth and were washed in the breakage buffer (10mM $NaPO_4$, pH7.5, 150mM NaCl, 5mM EDTA, 1mM PMSF) using an Amicon filtration apparatus. Approximately 1600g of cells were broken in a continuous-flow beadmill (Dynomill) in 6.4 liters total volume of buffer. The resultant cell lysate was centrifuged at 7700 x g; the supernatant from this spin was adjusted to pH 5, and subsequently centrifuged at 17,000 x g. The supernatant of this spin was adjusted to pH7 and filtered through two 0.45 μm filters into sterile polystyrene containers. The amount of p24 gag in the final sample was estimated by Western blot to be approximately 20-30 mg/l. The total protein concentration was determined by TCA Lowry to be 3.85 g/l. Thus, the final concentration of p24 gag in the sample was approximately 0.5-0.75% of total cell protein. We estimated the yield of the procedure to be approximately 33-50%, comparing the amount from hand breakage of a 100 OD aliquot of the final cell sample.

Example VII

Product Analysis

16

The amount of p24 gag in the final sterile filtrate was estimated by Western blot using a 35-kd fusion protein, PG2, from Centocor as a standard [see Ghrayeb et al (1986) DNA 5:93-99]; a 1:1250 dilution of rabbit antiserum to viral extracts from Bionetics Research, Inc. (Rockville, MD) and $^{125}$I-protein A (New England Nuclear) was used for detection.

The filtrate from the previous example was on a 10% polyacrylamide Laemmli gel following the protocol of Laemmli (UK) Nature 227:680-685 (1970). The protein was then electro-blotted from the gel onto a .45μm nitrocellulose filter by electrophoresis performed at 300m Amps constant current $1\frac{1}{2}$ -2 hours following the protocol of Towbin et al, Proc. Natl. Acad. Sci. USA, 76:4350-4354 (1979). The filter was then removed from the electroblot and incubated in Western Buffer for one hour. Antibody diluted in Western Buffer was then added and left overnight in the cold room on a shaker. The filter was washed 4 times for 15 minutes per wash in Western buffer. The filter was then incubated with $^{125}$I protein A for 45 minutes at .5μ Ci $^{125}$I-protein A/lane. The filters were again washed 4 times for 15 minutes per wash in Western Buffer and exposed to Kodak X-OMATAR film.

Immunoreactive bands migrating at approximately 80-90 kd, 39 kd, 27 kd, and 24 kd were detected on Western blots to P. pastoris strain G-GAG44. The three larger species are present in cell extracts of untransformed cells, and thus, apparently represent cross-reactions to antigens other than HIV gag p24. We estimate the concentration of p24 gag to be between 0.5 and 0.75% of the total cell protein.

The examples have been provided merely to illustrate the practice of the invention and should not be read so as to limit the scope of the inveniton or the appended claims in any way. Reasonable variations and modifications, not departing from the essence and spirit of the invention, are contemplated to be within the scope of patent protection desired and sought.

## Claims

1. A process for the production of the HIV 24 kDa gag protein comprising

a) transforming a methylotrophic yeast with at least one vector having at least one expression cassette containing a structural gene for HIV 24 kDa gag protein, operably linked to a regulatory region and a 3' termination sequence; and thereafter

b) culturing the resulting transformed yeast strain to obtain the production of said HIV 24 kDa protein.

2. The process of claim 1 wherein said vector is selected from plasmids and linear, integrative site-specific vectors.

3. The process of claim 2 wherein said linear integrative site-specific vector contains the following serial arrangement:

a) a first insertable DNA fragment,

b) at least one marker gene, and at least one expression cassette containing a structural gene for the HIV 24 kDa gag protein, operably linked to a regulatory region and a 3' termination sequence, and

c) a second insertable DNA fragment;

wherein the order of the marker gene and cassette of component (b) may be interchanged.

4. The process of claim 3 wherein the first insertable DNA fragment and the second insertable DNA fragment are derived from the DNA sequence of a gene isolated from Pichia pastoris and selected from AOX1, p40, DHAS and HIS4.

5. The process of claim 3 wherein said expression cassette comprises

a) a regulatory region selected from AOX1, p40, DHAS and HIS4, isolated from Pichia pastoris, acid phosphatase, galactokinase, alcohol dehydrogenase, cytochrome c, alpha-mating factor and glyceraldehyde 3-phosphate dehydrogenase isolated from Saccharomyces cerevisiae operably linked to

b) a structural gene for the HIV 24 kDa gag protein, operably linked to

c) a 3' termination sequence from Pichia pastoris selected from the 3' termination sequences isolated from the AOX1 gene, p40 gene, DHAS gene and HIS4 gene.

6. The process of claim 3 wherein said marker gene is selected from HIS4 and ARG4, isolated from Pichia pastoris, SUC2 isolated from Saccharomyces cerevisiae and G418$^R$ gene of Tn903 and Tn601.

7. The process of claim 3 wherein said vector comprises

a) a first insertable DNA fragment which is about one kilobase of the 5' AOX1 regulatory region isolated from Pichia pastoris operably linked to

b) a structural gene for the HIV 24 kDa gag protein operably linked to

c) the 3' termination sequence of AOX1 isolated from Pichia pastoris ligated to

d) at least one marker gene which is HIS4 isolated from Pichia pastoris ligated to

e) a second insertable DNA fragment which is about 0.85 kilobases of the 3′ AOX1 termination sequence.

8. A linear integrative site-specific vector comprising the following serial arrangement

a) a first insertable DNA fragment,

b) at least one marker gene and at least one expression cassette containing a structural gene for the HIV 24 kDa gag protein, operably linked to a regulatory region and a 3′ termination sequence, and

c) a second insertable DNA fragment;

wherein the order of the marker gene and cassette of component (b) may be interchanged.

9. The vector of claim 8, wherein said vector is defined as in any of claims 4 to 7.

10. Methylotrophic yeast transformed with at least one vector containing at least one expression cassette comprising a regulatory region operably linked to a structural gene for the HIV kDa gag protein, operably linked to a 3′ termination sequence.

11. The methylotrophic yeast of claim 10 wherein the yeast is Pichia pastoris.

12. The methylotrophic yeast of claim 11 wherein the yeast is Pichia pastoris strain GS115.

13. The methylotrophic yeast of any of claims 10 - 12 wherein said yeast is transformed with at least one linear integrative site-specific vector as defined in any of claims 3 to 9.

14. Pichia pastoris G-GAG44.

15. Pichia pastoris GS115 transformed as in claim 13 wherein said GS 115 is transformed with more than one copy of said linear integrative site-specific vector as defined in any of claims 3 to 9.

PARTIAL RESTRICTION MAP OF EcoR I/Cla I
FRAGMENT OF HIV ENCODING p24

*FIG. 1*

FIG. 2-1

FIG. 2-2

EcoR I

Pst I (240)

Hind III (533)

Hind III
(8826)

HIV p24

Bgl II
(8033)

5'AO

Eco RI (700)

3'AO

Cla I
(7903)

Cla I (1000)

Hind III (1006)

Ssp I
(7687)

~.95kb  .7kb

~.3kb

.35kb

HIS4

Pst I
(7126)

pSL44A
~8.83kb

f1-ori

⊖

~.426kb

2.7kb

Sal I (2502)

⊕

(6361) Ssp I

Nco I (3042)

.85kb

Bgl II
(5176)

3'AO

*FIG. 3*